# EUROPEAN PATENT SPECIFICATION

(11) **EP 2 958 037 B1**
(45) Date of publication and mention of the grant of the patent: **14.08.2019**
(21) Application number: 15171520.8
(22) Date of filing: 10.06.2015
(51) Int. Cl.: G16H 10/60, G06F 16/00, G06F 11/07, G06F 9/54, G06F 16/215

(54) **DATA COLLECTION AND CLEANING AT SOURCE**
DATENSAMMLUNG UND REINIGUNG AN DER QUELLE
COLLECTE ET NETTOYAGE DE DONNÉES AU NIVEAU DE LA SOURCE

(30) Priority: 17.06.2014 US 201414307235
(43) Date of publication of application: 23.12.2015
(73) Proprietor: IQVIA Inc., Parsippany, NJ 07054 (US)
(72) Inventor: FLOREZ, Elkin, Richmond Hills, Ontario L4C 0T8 (CA); BLAIR, Charles, Toronto, Ontario M2N 6M4 (CA)
(74) Representative: FRKelly

(56) References cited:
- EP-A1- 2 590 124
- US-A1- 2006 247 944
- US-A1- 2008 086 502
- US-A1- 2009 018 996
- US-A1- 2009 182 580
- US-A1- 2010 011 342
- US-A1- 2011 047 535

## Description

### BACKGROUND

### Field of the Invention

Embodiments of the present invention generally relate to data collection, and, in particular, to a system and method for defining data structure dynamically and detecting erroneous data collection closer to a source of the data, and correcting the erroneous data or limiting its further use.

### Description of Related Art

Mobile health ("mHealth") is a term for medical and public health practice supported by communication terminals such as mobile phones, patient monitoring devices, personal digital assistants (PDAs), and other mobile or wireless devices. mHealth involves the use of voice and short messaging service (SMS) as well as more complex technologies such as mobile data communication systems (e.g., 3G, 4G, 4GLTE, etc.), global positioning systems (GPS), and Bluetooth technology.

A mobile application (or mobile app) is a software application designed to run on smartphones, tablet computers and other mobile devices. Some mobile apps are used to deliver sensitive personal information such as health care information to consumers, or to gather and send health status information from a consumer to a health care provider. Not all mobile apps that relate to the exchange of sensitive personal information, for example those that have been developed in healthcare, are widely available to consumers. Some of the most advanced medical apps are not necessarily designed to target general consumers. Some mobile apps have been designed for healthcare practitioners, others are for patients but require a prescription, and others are intended for only a small subset of patients. As used herein, the term "mobile app" or "mobile application" may include an application that executes on a PC (e.g., desktop, tower, laptop, netbook, etc.) or other general-purpose consumer-computing device, without limitation to a mobile device unless mobility provides a stated benefit or unless otherwise clearly restricted by the context of usage.

An information system, such as a system used for health care, may generate and use electronic data collected at multiple sources such as mobile applications, desktop applications, web applications, and so forth. The electronic data is inherently subject to data entry error, regardless of whether the data is entered manually or by sensor readings. For instance, manually-entered electronic data may include typing errors (e.g., mis-typed digit, transposed digits, missing digit, double key strike, non-digit keystroke, misplaced decimal point, etc.). Electronic sensor readings may suffer from sensor failure (e.g., outputting all zeros or some other invalidity code), sensor misplacement or dislodging, communication line failures, undue influence from the environment (e.g., temperature effects, vibration or RF interference), time base inaccuracy, etc. Data entry errors need to be corrected or removed so that analysis and decisions are made using only trustworthy data, i.e., electronic data that is substantially error-free, or having negligible residual errors. Data should also be meaningful, i.e., be sufficiently thorough or comprehensive, such as by including a statistically significant sampling, or a complete cycle of an underlying process, or the like as the situation dictates. Trustworthy and meaningful electronic data, i.e., information that is reliable enough and complete enough to be acted upon, is actionable information that may be analyzed and used to make and support decisions.

Presently known processes for transforming data to actionable information require multiple steps, is expensive and time consuming and often results in incomplete or inaccurate data. A first problem is that it is very expensive to transform data to actionable information when (as in most instances) at least some of the data is invalid in some way, such as by being incomplete, inaccurate or containing garbage elements. The collected data is sent to a cleaning site, e.g., a processing system at which manual or semi-automated systems may be used to detect suspected invalid data, and to flag, to correct, and/or to remove the suspected invalid data. When data with these types of deficiencies goes through a cleaning process, the resulting information may still contain gaps, inaccuracies and/or otherwise invalid information.

A second problem is that a conventional data cleaning process is time consuming and is usually done using a batch process and the source of the data is not available to provide corrections. For instance, the data first has to be collected, then the data is sent to a server for cleaning. A batch process is used so that the cleaning process can be done in off-periods when computing capacity is less likely to be needed for other tasks such as user support, or so that the cleaning jobs can be submitted without the source of the information attending further to the process.

A third problem of the conventional art pertains to applications that can process structured data and perform simple validation of data its source. Such applications typically use a regular expression (as known in its computer science sense) to prevent a user from entering erroneous data, or to provide an information limit that prevents the user from entering unrealistic information (e.g., blood pressure above 500 mmHg). The problem with this conventional art is that more complex inter-related or dependent data scenarios require other types of validations, which are not easily managed by a regular expression. For complex data validation, applications would need to send the data collected to a back end server for quality control processing and management, which would be a complicated and time consuming process.

A fourth problem of the conventional art is that application developers usually hardcode specific data structures or validation rules (also referred to as data consistency rules) in their applications for specific use cases. Later, if the data structure changes or if the data validation rules change, the application source code must be changed and a new version of the application distributed to the user base. The user will be required to download and install a new version of the application to access the latest changes. A user who continues to use an older version of the application may introduce erroneous data or data that does not otherwise pass the most recent data validation rules.

The known background art cannot update their data structures and quality rules on the fly. The known background art requires changes to the source code of the application for each update, and users are required to download each updated version, which is awkward and inconvenient, and potentially prone to continued problems with data quality if users are able to defer or decline the download and installation of updated versions of the application.

Furthermore, the known background art typically performs data quality processing (e.g., data cleaning) at the server side, especially for complex data. Thus, end user data is collected at the application and then sent to the application server for batch processing. This is time consuming, costly and does not lend itself well to an interactive model.

EP 2 590 124 relates to a knowledge-driven data quality solution that is based on a rich knowledge base. The data quality solution can provide continuous improvement and can be based on continuous (or on-going) knowledge acquisition. The data quality solution can be built once and can be reused for multiple data quality improvements, which can be for the same data or for similar data.

US 2006/247944 relates to the provision for scalable cleansing and value enhancement of data in the context of a multi-source multi-tenant data repository. The source data comes from multiple sources and on multiple topics. Evolutionarily tracked source data tags are used to hold tracking information reflecting the nature and sources of each change to the data, as it is affected during the various stages of data processing. The stages of processing include validation, normalization, single-source cleansing and cross-source processes. Various rules are applied during these stages, and evolutionarily tracked source data tags are used to record sources and agents of all changes to the data. As information is processed, transformed, and added to the repository, corresponding evolutionarily tracked source data tags are stored in association with the various information elements. The information contained in these tags can be used to enforce data entitlements in a multi-tenant data repository environment.

US 2009/018996 relates to a method which involves receiving a dataset in an analytic platform, the dataset including fact data and dimension data for a plurality of distinct product categories. Storing the data in a flexible hierarchy, the hierarchy allowing the temporary fixing of data along a dimension and flexible querying along other dimensions of the data. Pre-aggregating certain combinations of data to facilitate rapid querying, the pre-aggregation based on the nature of common queries. Facilitating the presentation of a cross-category view of an analytic query of the dataset. The temporarily fixed dimension can be rendered flexible upon an action by the user.

US 2009/182580 relates to a system and method for automatedly aggregating medically related data and disseminating different sets of the aggregated data to two or more different members of a medical care provider. Some embodiments automatedly and intelligently disseminate the aggregated data such that the data that is written once to the data storage solution is usable for a multitude of purposes within the functions of the medical care provider. Some embodiments intelligently disseminate the aggregated data by disseminating only relevant sub-components of the data to different members of the medical care provider based on the needs of the members such that different members receive different subsets of the data.

Therefore, a need exists to improve data validation closer to the source of the data, in order to provide more trustworthy and actionable information to support decision-making, data cleaning at the source and ultimately to provide improved and customer satisfaction.

### SUMMARY

Embodiments in accordance with the present disclosure relate to a mobile device to cleanse data as defined in claim 1 and a method to cleanse data as defined in claim 11. Advantageous features are defined in the dependent claims.

### BRIEF DESCRIPTION OF THE DRAWINGS

The above and still further features and advantages of the present invention will become apparent upon consideration of the following detailed description of embodiments thereof, especially when taken in conjunction with the accompanying drawings wherein like reference numerals in the various figures are utilized to designate like components, and wherein:
FIG. 1 illustrates an exemplary configuration file in accordance with an embodiment of the present invention;
FIG. 2 illustrates at a relatively high modular level of abstraction a system in accordance with an embodiment of the present disclosure;
FIG. 3A illustrates at a relatively high hardware level of abstraction a system in accordance with an embodiment of the present disclosure;
FIG. 3B illustrates at a relatively high hardware level of abstraction a system in accordance with another embodiment of the present disclosure;
FIG. 4 illustrates a process to use a system by a server, in accordance with an embodiment of the present disclosure; and
FIG. 5 illustrates a process to use a system by a mobile device, in accordance with an embodiment of the present disclosure.

The headings used herein are for organizational purposes only and are not meant to be used to limit the scope of the description or the claims. As used throughout this application, the word "may" is used in a permissive sense (*i*.*e*., meaning having the potential to), rather than the mandatory sense (*i*.*e*., meaning must). Similarly, the words "include", "including", and "includes" mean including but not limited to. To facilitate understanding, like reference numerals have been used, where possible, to designate like elements common to the figures. Optional portions of the figures may be illustrated using dashed or dotted lines, unless the context of usage indicates otherwise.

### DETAILED DESCRIPTION

The disclosure will be illustrated below in conjunction with an exemplary communication system. Although well suited for use with, e.g., a system using a server(s) and/or database(s), the disclosure is not limited to use with any particular type of communication system or configuration of system elements. Those skilled in the art will recognize that the disclosed techniques may be used in any communication application in which it is desirable to provide more actionable data collection.

The exemplary systems and methods of this disclosure will also be described in relation to software, modules, and associated hardware. However, to avoid unnecessarily obscuring the present disclosure, the following description omits well-known structures, components and devices that may be shown in block diagram form, are well known, or are otherwise summarized.

In the following detailed description, numerous specific details are set forth in order to provide a thorough understanding of embodiments or other examples described herein. In some instances, well-known methods, procedures, components and circuits have not been described in detail, so as to not obscure the following description. Further, the examples disclosed are for exemplary purposes only and other examples may be employed in lieu of, or in combination with, the examples disclosed. It should also be noted the examples presented herein should not be construed as limiting of the scope of embodiments of the present invention, as other equally effective examples are possible and likely.

As used herein, the term "module" refers generally to a logical sequence or association of steps, processes or components. For example, a software module may comprise a set of associated routines or subroutines within a computer program. Alternatively, a module may comprise a substantially self-contained hardware device. A module may also comprise a logical set of processes irrespective of any software or hardware implementation.

As used herein, the term "gateway" may generally comprise any device that sends and receives data between devices. For example, a gateway may comprise routers, switches, bridges, firewalls, other network elements, and the like, any and combination thereof.

As used herein, the term "transmitter" may generally comprise any device, circuit, or apparatus capable of transmitting a signal. As used herein, the term "receiver" may generally comprise any device, circuit, or apparatus capable of receiving a signal. As used herein, the term "transceiver" may generally comprise any device, circuit, or apparatus capable of transmitting and receiving a signal. As used herein, the term "signal" may include one or more of an electrical signal, a radio signal, an optical signal, an acoustic signal, and so forth.

As used herein, the term "application container" may generally refer to a mobile application that can host and support the usage of several application configurations. Each configuration describes the GUI appearance, application flow, logic and data relevant to an application. The container may start with one configuration that is identified as the first configuration. The first configuration may allow a user to select the other configurations to use.

The term "computer-readable medium" as used herein refers to any tangible, non-transitory storage and/or transmission medium that participates in storing and/or providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, RAM, PROM, EPROM, FLASH-EPROM, solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored.

Embodiments in accordance with the present disclosure provide and utilize a set of generic configurable processes, implemented as application program interface (API) modules, to enable the management of unknown data structures and validation rules at run time. Application programs that incorporate the embodiments will accept documents or data only if they are compliant with a data structure and a validation rule set defined during the initialization process.

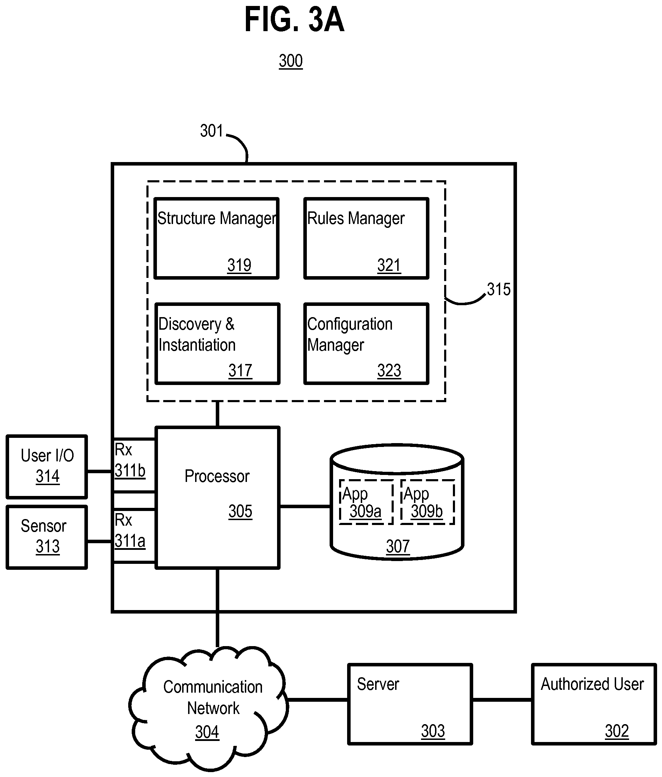

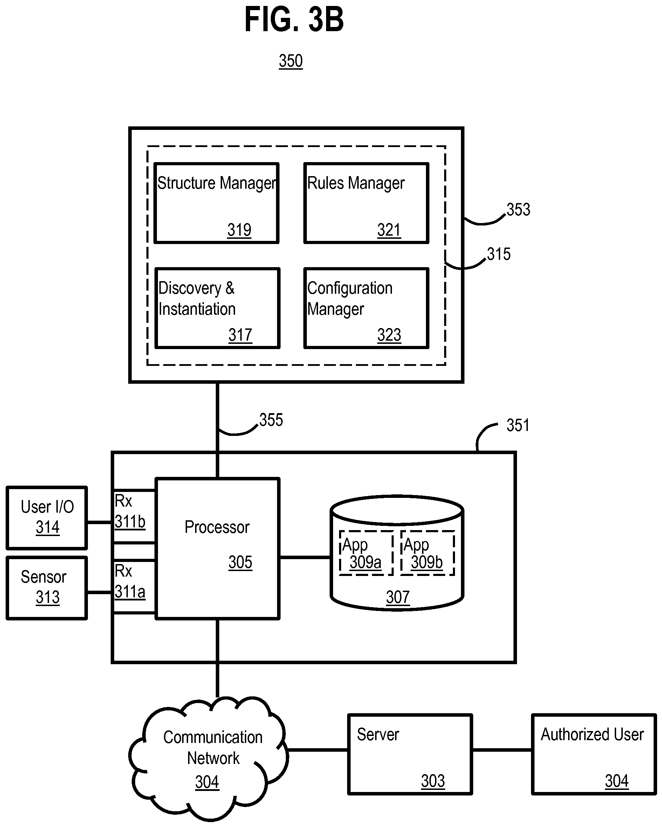

Embodiments in accordance with the present disclosure may be adapted to execute on a variety of target computing platforms (e.g., smart phone, tablet, laptop, netbook, other mobile device, desktop, etc.), which are described below in greater detail in the context of FIGS. 3A and 3B. Generic configurable processes such as a data API, a rules API, a rules engine, and so forth, may be written in native code for the target computing platforms. Native code is known as computer programming code that is compiled to run directly with a particular processor and its set of instructions (e.g., machine code). The generic configurable processes may be packaged for distribution to the target computing platform as components of a generic application ("app") container, new data structures and rules, or changes to existing data structures and rules. The generic configurable processes may be implemented without a need to change or update the source code of the generic app container.

Embodiments in accordance with the present disclosure enable different applications, using a shared type of data structure, to collect structured information sets at the source. The structured information sets will be of higher quality (e.g., more trustworthy and meaningful) and well organized (e.g., possessing a similar and consistent structure), while reducing the number of steps required to cleanse the collected data, in order to achieve actionable information from the data collected. Cleansing of data refers to the identification and correction or removal of untrustworthy data, e.g., data that includes significant errors or data gathered under unknown conditions when knowledge of those conditions would be significant for purposes of normalization, compensating, correcting, or the like. An example of errors that may not be significant are measurements that vary within the usual tolerance or accuracy of a sensor or a timer, or statistical sampling error. An example of errors that may be significant are measurements that include a relatively large variation or systematic bias (relative to the usual tolerance, accuracy or sampling error) due to influence from the environment (e.g., temperature effects, vibration or RF interference). It can be difficult to distinguish between errors that should be cleansed and true changes in an underlying process being monitored. Embodiments, by reducing the need to cleanse data, save resources while improving the quality of the information produced.

With respect to a drawback of the background art discussed above, i.e., that it is very expensive to transform data to actionable information when (as in most instances) at least some of the data is invalid in some way, embodiments in accordance with the present disclosure may be used by multiple information sources (e.g., smart phone, tablet, laptop, netbook, other mobile device, desktop, etc.) in order to collect data at the data source more efficiently and consistently. Embodiments achieve this objective by using data structures, rules and quality control configurations to ensure that the collected data conforms to what is expected, providing an enforced consistency. Because this consistency occurs when the data is entered, users can be alerted to missing information, inaccurate information, inconsistent information or information that does not conform to the expected format. Determining consistency may involve the use of historical data and data from other sources. Users are then able to correct the information before it is submitted to a server for further usage and analysis.

With respect to another drawback of the background art discussed above, i.e., that a conventional data cleaning process is time consuming and is usually done using a batch process, embodiments in accordance with the present disclosure enables an application (e.g., an app related to mHealth) to manage the entry of information during a user interaction with the application. By this process, information collection and management may be interactive, transactional and fast.

Furthermore, because of the transactional and interactive characteristics of applications, embodiments permit information to be processed substantially in real time. Embodiments facilitate being able to update and manage statistics or reports almost instantaneously once the information is received by a server, because the data is deemed to be actionable. This may be very valuable for many types of applications where time may be of the essence (e.g., applications that evaluate the effects of advertising campaigns, applications that detect acute medical conditions, etc.).

With respect to another drawback of the background art discussed above, i.e., that more complex inter-related or dependent data scenarios require types of validations that are too complex to specify with a regular expression, or that are not easily managed by a regular expression, embodiments enables both structured data processing and data quality control of substantially all types of data sets (e.g., simple or complex data sets) at source, without a need to construct validation at the server side when the source of that data may not be available anymore.

With respect to another drawback of the background art discussed above, i.e., that application developers usually hardcode specific data structures or validation rules in their applications for specific use cases, embodiments allow a developer to develop a software app without having to worry about the format of the data structures and validation rules, or having to worry about changes to the data structures and validation rules, or having to worry about management of data quality. Embodiments help ensure that the software app can be compliant with predefined standards or initiatives, such as for mHealth uses, by providing embodiments to manage information, provided by users, at the source of the information. Changes to the data structure of the information to be gathered (e.g., field definition) or quality requirements of the entered information (e.g., level of, or degree of error checking and consistency checking of numeric information; calculating interrelationships between data) will not require changes to source files of the software app. Embodiments may manage data structures and validation rules set (including changes thereto) by use of formal change configuration and control methods. Embodiments will tend to reduce the complexity of software app development, such as for mHealth applications, because the data structures and validation rules-set configuration will be managed in a consistent manner and accessible to software developers.

Embodiments in accordance with the present disclosure provide a flexible framework for collecting data and transforming that data to actionable information, at the source. Flexibility includes being able to reconfigure on-the-fly formatting and consistency requirements related to the collected data, i.e., in real time without needing to redistribute or restart the application program. The data structures and validation rules required for specific disease type are important, and may change over time with the advancement of medical knowledge. At a particular point in time, state of the art medical knowledge may deem certain factors or behavior to be markers of elevated risk for a disease. For example, four cup of coffee or two glasses of red wine per day may be deemed healthful one year, but in a later year be deemed by new studies to be unhealthy or to have no statistically meaningful effect. As a result, over time it may be desirable to have a medical app collect different patient data over time, or to analyze collected data in different ways over time. Embodiments can adapt to such advancements in medical knowledge by permitting and facilitating changes to the required data structures and validation rules in order to continue to gather actionable information, without a need to redistribute the medical app itself.

Embodiments described herein are not limited to use in a medical context, but may be useful across a variety of contexts or industries. Embodiments facilitate consistent data collection from similar applications, even when written by different developers, and consistent management of the similar applications.

Although other technologies (e.g., XML and XML schema) may offer limited flexibility in the display of information, these technologies are deficient in offering a flexible ability to check the data at the source for accuracy and consistency, or to manage integrated data structure and validation rules engines. Embodiments facilitate enhanced error and consistency checking.

Embodiments in accordance with the present disclosure may use a data API and a validation rules engine that are coded once at the native level but may be reconfigured at run time to produce actionable information relevant to the purpose of a software application. Embodiments thus allow the software application to support the required data structures and the validation rules set for data consistency, data quality and process flow, to produce actionable information.

Another advantage of embodiments in accordance with the present disclosure is that changes to data structures and validation rules may be implemented at a configuration level rather than at an application source code level. For example, configuration-level implementations may rely upon an application program reading a configuration file upon initialization and, optionally, periodically or other additional times thereafter. Changes to data structures and validation rules may be coded into the configuration file. Therefore, on-the-fly changes (i.e., real time changes) that impact the application workflow, user experience (UX) or user interface (UI), data type or validation rules may be supported without causing a need to change, update, or restart the software application.

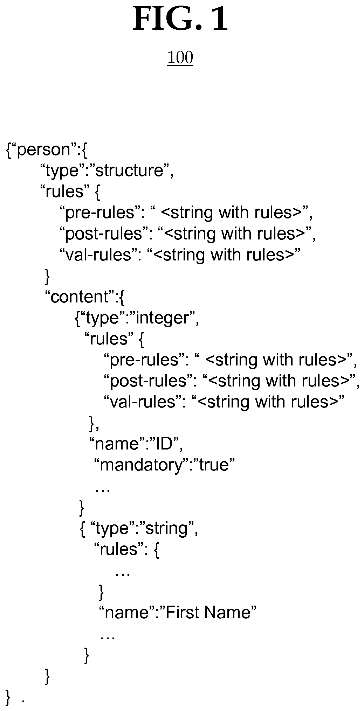

A configuration file may be provided by the use of a language that can be implemented in different ways, such as using JavaScript Object Notation (JSON) or Extensible Markup Language (XML), or substantially any other specific implementation. The configuration file may comprise a data structure description that contains a specification of hierarchy, and having the data fields and structure markers associated with validation rules. An exemplary configuration file using JSON is illustrated in FIG. 1. Although FIG. 1 illustrates one example of how a configuration file may look like, other implementations of a configuration file are contemplated.

From an implementation perspective, the configuration would be managed by the platform and the data API and validation rules engine components would be embedded in an app container, e.g., by use of configuration data, initialization file, and the like. A data API and a validation rules engine read the configurations and manage the required behavior accordingly. An application ("app") container may be a mobile application that can host and support the usage of several application configurations. Each configuration describes the GUI appearance, application flow, logic and data. The container may start with one configuration that is identified as the first configuration. The first configuration may allow a user to select the other configurations to use.

For example, suppose that a dosage of a drug is specified in units of "IU." Further suppose that a user supplies measurements of the drug (e.g., for monitoring or enforcing compliance) in units of "mgs." In such a situation, the validation rules engine will catch this error.

However, further suppose that at a later time there is a need or desire to update the validation rules in order to accept measurement in units of "mgs." For systems of the known art, such an update to the validation rules cannot be made without a need to update the application source code, and then distribute compiled, or run-time versions of the software application to users. This forces the users to update their devices with the new updated version of the software application. Such an update is particularly inconvenient for mobile users. For example, inconvenience may arise from mobile users exceeding their monthly data transfer limit, or mobile users may become inured to frequent updates, or because of a delay in availability of the software application caused by a need to submit the software application to validation testing and approval before it is made available for download by users. There may be a delay between the time that a software update is ready, and the time that a user permits or chooses to update the software application.

Further, in the absence of the embodiments described herein, the data collected from users would still need to be reviewed, normally by a person or a system, and cleaned if any deficiency is present. All of this processing would likely be done in batch mode and would take a long time. Also, this processing may be error-prone because cleaning the data is at least partially a manual process, and thus subject to attendant manual errors.

In contrast, and upon such a request to update the validation rules, embodiments may reconfigure the software app without having to distribute an update of the software app itself, to end users such that end users can then enter a dosage of their medication in units of "mgs." Similarly, the validation rules set may also be updated to address changes in the dosage of a drug, the number of repetitions, the validation of drug interactions or identification of harmful interactions, and so forth.

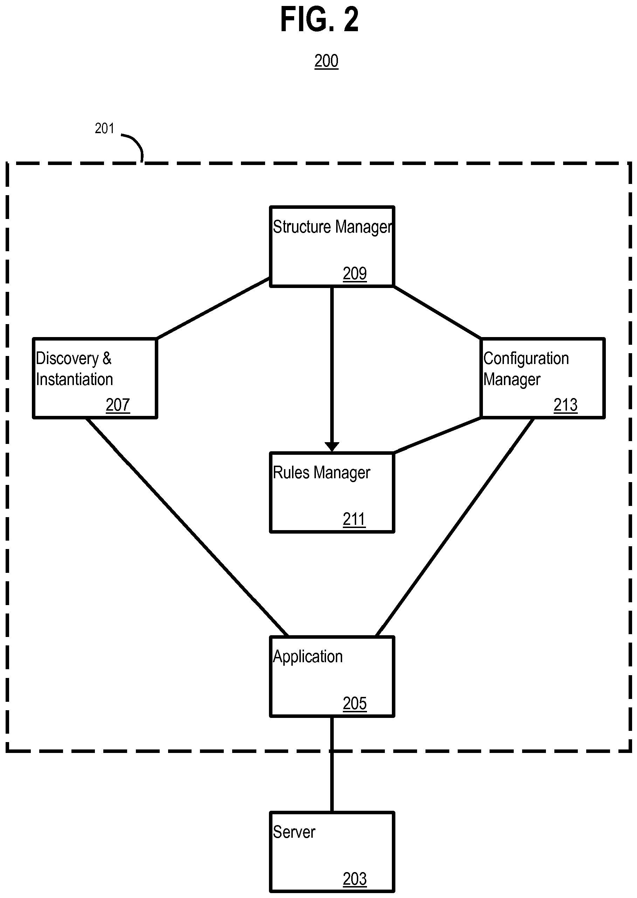

FIG. 2 illustrates at a relatively high modular or functional level of abstraction a system 200 in accordance with an embodiment of the present disclosure. System 200 includes a mobile device 201 in communication with a server 203. Mobile device 201 may include an application program 205 that functions to exchange information and/or commands with server 203, e.g., to receive commands from server 203 regarding control of data measurements, and to communicate measured data from application program 205 to server 203.

Mobile device 201 may further include a discovery and instantiation module 207. The discovery and instantiation module 207 may be invoked by application program 205 when application program 205 needs to take a measurement. The instantiation functionality of the discovery and instantiation module 207 is used by the application program 205 to build in memory all the objects required by the API (i.e., the embodiments) to work. The application program 205 will use the discovery functionality of the discovery and instantiation module 207 to learn the data structure available according to the configuration provided to the API. The application program 205 can use the description of the information provided by the discovery and instantiation module 207 to know the data that needs to be requested from or provided to the user. The application program 205 may also use the API provided by the discovery and instantiation module 207 to provide the corresponding data according to the data configuration. The discovery and instantiation module 207 will delegate the data validation to the structure manager module 209 described below.

Mobile device 201 may further include a structure manager module 209 that is in communicative contact with discovery and instantiation module 207. The discovery and instantiation module 207 may delegate to structure manager module 209 certain tasks related to data validation. For example, when discovery and instantiation module 207 has one or more measurements that need to be validated, discovery and instantiation module 207 may send the data to structure manager module 209 with a request to validate the data. Data validation may include, e.g., ensuring that the particular data instantiation being verified is within limits (e.g., min and max value limits) and contains no data entry errors (e.g.: a measurement having nonnumeric characters, missing or additional decimal points, or a negative sign if not appropriate for the data; invalid characters in an email address such as not having exactly one "at symbol"; invalid characters in a postal address; and so forth), or that the data otherwise conforms to a required structure (e.g., that a blood pressure measurement include both a systolic and diastolic measurement). Data validation performed by the structure manager module 209 may be considered to be self-referential validation, to the extent that the validation examines the particular data in isolation, without necessarily comparing the data to be validated with other data. Discovery and instantiation module 207 may also send information that identifies the data, so that structure manager module 209 can identify the correct set of validation rules to apply to the data.

Mobile device 201 may further include rules manager module 211 that is in communicative contact with structure manager module 209. Structure manager module 209 may delegate to rules manager module 211 certain tasks related to rules validation. For example, when discovery and instantiation module 207 has one or more measurements that need to be validated, part of the validation may involve a more comprehensive examination of the measurements to be validated, in order to determine whether the measurement conforms to rules related to relationships of this measurement with other measurements of the same parameter or a different parameter. For example, a measurement from a continuous glucose monitor may be examined to see if it has not changed for a predetermined number of consecutive readings (which may be indicative of a sensor problem) or if the data does not follow expected diurnal patterns or does not follow patterns related to other, external events such as eating meals. Data validation performed by the rules manager module 211 may be considered to be relative validation, to the extent that the validation examines the particular data by comparing the data to be validated with other data.

Mobile device 201 may further include a configuration manager module 213 that is in communicative contact with application program 205. Configuration manager module 213 provides functions that can be used to facilitate changing the data structure or the validation rules. For example, it may be determined that the data structure of a series of measurements needs to be changed (e.g., by changing the min/max limits of acceptable readings), or that the rules need to be changed (e.g., including a diurnal component to the acceptable ratio between a systolic and diastolic blood pressure measurement). The requested change may be either inputted to application program 205 by a user (e.g., via server 203), or may be algorithmically determined by application program 205 (e.g., as an automated attempt to reduce false alarms). It will be determined whether the change involves a change to data validation, to rule validation, or to both. In some embodiments, this determination may be made by application program 205 and communicated to configuration manager module 213 along with the change itself. In other embodiments, the determination may be made alone by configuration manager module 213 from the change, without a need for application program 205 to make this determination.

Configuration manager module 213 is in communicative contact with structure manager module 209 and with rules manager module 211. Configuration manager module 213 may recognize (either by being informed or by its own determination) whether the request involves a change to data structure or to rule validation. If the change relates to data structure, configuration manager module 213 will communicate with structure manager module 209 in order to update the required data structure. If the change relates to rule validation, configuration manager module 213 will communicate with rules manager module 211 in order to update the required rule validation.

When data validation takes place, one of two scenarios may occur: First, the information may be in accordance with the validation rules; or second, the information may not be in accordance with the validation rules. In the second case, the embodiments notify the application program 205 that something was wrong, and will also notify the application program 205 what was wrong. Embodiments may return one or more of several types of notification to the application program 205, for example, in order to indicate severity (e.g., errors, warnings), to indicate a suspected cause of the error, and so forth.

FIG. 3A illustrates at a relatively high hardware level of abstraction a system 300 in accordance with an embodiment of the present disclosure. System 300 may be useful to illustrate a workflow sample of data collection and cleaning at the source.

System 300 includes a mobile device 301 in communication with a server 303 through communication network 304. Mobile device 301 may include a communication interface (e.g., wired Ethernet, wireless Wi-Fi or Bluetooth transceiver, etc.) in order to communicate with communication network 304, using hardware and protocols as known to persons of skill in the art. Mobile device 301 may include a processor 305 coupled to a memory 307. Memory 307 may be configured to store one or more application (or app) programs 309a, 309b, etc. that, when executed by processor 305, functions to exchange information and/or commands with server 303, e.g., to receive commands from server 303 regarding control of user input such as data measurements or other typed information, and to communicate measured data from processor 305 to server 303. An individual but nonspecific application program may be referred to as application program 309. An authorized user 302 is allowed to communicate with server 303 and to configure server 303, e.g., by defining rule sets, data structures, and other descriptions to indicate valid data. Such configurations in turn will be communicated to mobile device 301.

The communication network 304 may be packet-switched and/or circuit-switched. An exemplary communication network 304 includes, without limitation, a Wide Area Network (WAN), such as the Internet, a Public Switched Telephone Network (PSTN), a Plain Old Telephone Service (POTS) network, a cellular communications network, or combinations thereof. In one configuration, the communication network 304 is a public network supporting the TCP/IP suite of protocols.

Mobile device 301 may further include a receiving interface circuit, e.g., receiver 311a, that is configured to receive measurements from a sensor 313. The measurements may include readings or other information that is to be cleaned. Receiver 311a may be coupled to processor 305 in order to pass measurements from sensor 313 to processor 305.

Mobile device 301 may further include a receiving interface circuit, e.g., receiver 311b, which is configured to receive data from a user input/output (I/O) device 314 such as a keyboard, touch-screen interface, microphone for voice recognition, and so forth. The data may include numeric data or other information that is to be cleaned. Receiver 311b may be coupled to processor 305 in order to pass user I/O from I/O device 314 to processor 305.

Processor 305 may be configured to access app container 315. App container 315 includes software code used to carry out the methods described herein, along with a set of APIs used by a calling (e.g., parent) process to access the software. The access may be by way of function calls, event handlers, or the like, called by or otherwise communicating with application program 309. For example, an event handler may detect the presence or availability of new data read through receiver 311a or 311b, and then invoke an API in app container 315 in order to process the data. After the data is processed, it may be supplied to application program 309 for further processing or analysis. App container 315 may be hosted in a memory within mobile device 301 (e.g., including memory 307 or a different memory), or may be hosted remotely and accessible to mobile device 301 over a communication link as illustrated in FIG. 3B, or some combination of the two.

App container 315 may include APIs such as discovery and instantiation API 317, structure manager API 319, rules manager API 321, and configuration manager API 323. Discovery and instantiation API 317 provides programmatic access to discovery and instantiation module 207. Structure manager API 319 provides programmatic access to structure manager module 209. Rules manager API 321 provides programmatic access to rules manager module 211. Configuration manager API 323 provides programmatic access to configuration manager module 213.

Server 303 may be configured to use configuration manager module 213, through processor 305 and configuration manager API 323, in order to change rules contained within structure manager module 209 and/or rules manager module 211. The changes may include, for example, changes to the rules set or changes to the data structure description.

FIG. 3B illustrates at a relatively high hardware level of abstraction a system 350 in accordance with an embodiment of the present disclosure. In comparison to system 300 of FIG, 3, system 350 stores app container 315 in a memory 353 that is remote from mobile device 351, but is in communication contact with mobile device 351 via communication link 355. Mobile device 351 may include a communication interface (e.g., wired Ethernet, wireless Wi-Fi or Bluetooth transceiver, etc.) in order to communicate with communication network 304 and/or memory 353, using hardware and protocols as known to persons of skill in the art. Other elements of system 350 have been described in the context of like numbered elements of system 300.

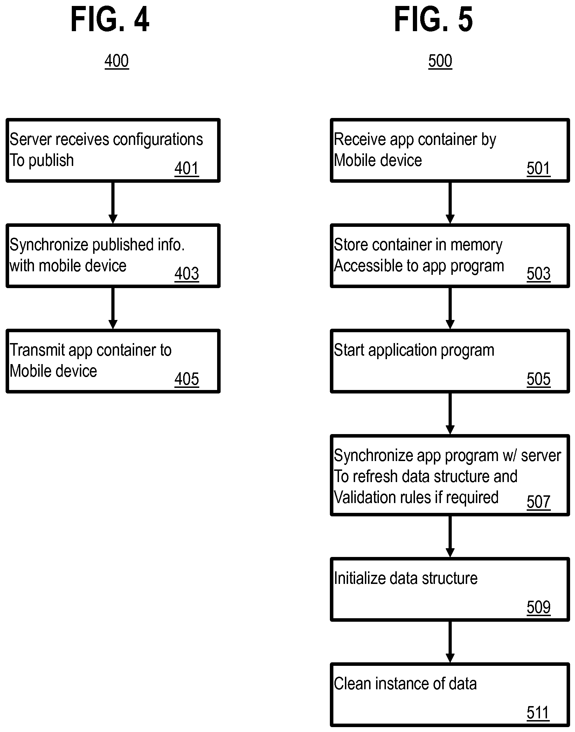

FIG. 4 illustrates a process 400 to use system 300, in accordance with an embodiment of the present disclosure. At least some steps of process 400 may be practiced by server 303. Process 400 begins at step 401, at which an authorized user 302 may publish to the server 303, and the server 303 may receive, configurations for one or more of: (A) a rules set to be implemented by rules manager module 211; (B) a data structure description to be implemented by structure manager module 209; and (C) application program 309.

Process 400 next transitions to step 403, at which published structures and rule configurations may be synchronized with mobile device 301. Such synchronization may take place at substantially any point in time. An update to the mobile device after application program 309 is already running may occur, for example, if new medical research indicates that additional types of data should be collected or if additional data-checking of the collected data should be performed (e.g., to compensate for a previously unrecognized diurnal bias, or to compensate for new medications that the patient may be taking, and so forth). In one embodiment, server 303 may then assemble the published information into an app container for transmission to mobile device 301. In another embodiment, the published information may be transferred to mobile device 301 during a synchronization process such as process 500 (described below). Transferring information to mobile device 301 may be repeated for additional data or sets of data that are to be collected. Some data (e.g., a blood pressure measurement) may include components (e.g., systolic and diastolic measurements) that may have some value individually, but the data may have more value as a set that includes all components measured at approximately the same time. Sets of data may also include measurements of a same process but repeated over time (e.g., a pulse rate monitor during exertion, or a glucose monitor during meals).

Process 400 next transitions to step 405, at which server 303 will then transmit the published information to a memory storage that is accessible to application programs 309. In some embodiments, the memory storage may be within mobile device 301 as illustrated in FIG. 3A. In other embodiments, the memory storage may be external to mobile device 351, as illustrated in FIG. 3B. At the conclusion of process 400, control may transfer to process 500 of FIG. 5.

FIG. 5 illustrates a process 500 to use system 300, in accordance with an embodiment of the present disclosure. At least some steps of process 500 may be practiced by mobile device 301 and/or application program 309 within mobile device 301. Although process 500 is described in reference to storing app container 315 within mobile device 301, persons of skill in the art will recognize how to adapt process 500 to include storage outside of mobile device 301. Process 500 begins at step 501, at which a mobile device 301 receives app container 315 from server 303. App container 315 includes APIs used to access software in app container 315.

Process 500 next transitions to step 503, at which the container received at step 501 is stored in a memory accessible to application program 309 within mobile device 301.

Process 500 next transitions to step 505, at which an application program 309 starts up. Individual APIs within app container 315 are available for use by application programs 309 in order to create data structures, to create and update data, and to validate data in order to help ensure higher quality data. For example, a software developer of as application program 309 or an authorized user 302 could use the data structure description and rules set, accessible through structure manager API 319 and rules manager API 321, in order to develop or use a specific type of mHealth application (e.g., a diabetes app). Usage of app container 315 helps ensure that application program 309 is reliably able to manage data consistency and data quality in the same manner as other, similar applications. The individual APIs form a library of functional interfaces that can be used by multiple application programs 309 in order to provide uniformity of user experience and reduce development time across application programs 309.

App container 315 may be a generic application (i.e., software to execute on mobile device 301, which is not directly related to data collection and cleaning at the source), which contains the configurable data API, rules manager API 321 and (optionally) rules components. Rules components refer to actual rules (e.g., systolic < 190), whereas the rules API refers to the set of methods used to perform the validation. The app container does not need to have prior knowledge of the content or configuration of the data structure and associated rules that are enforced by the APIs, i.e., there is no hard coding of the data structure and associated rules within application program 309. This allows application program 309 to be designed and used to manage substantially any type of application configuration.

Process 500 next transitions to step 507, at which application program 309 may synchronize itself with server 303 in order to refresh data structure and validation rules used by application program 309. For example, refresh may be needed when application program 309 is first executed, if an app container 315 is not already resident within mobile device 301. If an app container 315 is already resident in order to service application program 309a, then it may not be necessary to install another app container 315 to handle application program 309b. If app container 315 is already resident, then application program 309 may access and update data structure configuration, rules set configuration and user data information for the application type of application program 309.

Process 500 next transitions to step 509, at which application program 309 may then initializes itself and initializes rules manager module 211 and structure manager module 209 with configurations received from server 303.

Process 500 next transitions to step 509, at which application program 309 may then execute normally, in accordance with the received configuration, and accepts information from a user, e.g., through sensor 313 or user I/O 314. After application program 309 creates objects of the data structure using discovery and instantiation API 317, the objects thereafter will be used to validate user information by use of structure manager API 319 and rules manager API 321.

When an updated data structure configuration or rules set configuration is delivered to mobile device 301, the structure manager API 319 and rules manager API 321 may be initialized to support a new behavior. Embodiments therefore provide high quality data collection at a source without changing the source code of an application. The only thing that needs to be changed is configuration information associated with the application.

Embodiments in accordance with the present disclosure are useful to data collection organizations that may otherwise expend a great deal of resources to clean data because the data collected at source is unreliable without practice of the embodiments described herein. Embodiments will help users reduce the cost of data cleaning and improve the consistency with which application programs that depend upon actionable data can collect and manage data at a data source for different information types (e.g., data types). For example, for some health care related applications, information is needed substantially in real time for analytics and patient engagement. Embodiments in accordance with the present disclosure may enable data to be collected at a source, which may provide a more streamlined process for making the data available.

Furthermore, embodiments in accordance with the present disclosure facilitate managing and tracking application usage and health information for improved patient care across multiple apps and multiple user groups.

Embodiments of the present invention include a system having one or more processing units coupled to one or more memories. The one or more memories may be configured to store software that, when executed by the one or more processing unit, allows practice of the invention, at least by use of processes described herein, including at least in FIGS. 4-5, and related text.

The disclosed methods may be readily implemented in software, such as by using object or object-oriented software development environments that provide portable source code that can be used on a variety of computer or workstation platforms. Alternatively, the disclosed system may be implemented partially or fully in hardware, such as by using standard logic circuits or VLSI design. Whether software or hardware may be used to implement the systems in accordance with various embodiments of the present invention may be dependent on various considerations, such as the speed or efficiency requirements of the system, the particular function, and the particular software or hardware systems being utilized.

While the foregoing is directed to embodiments of the present invention, other and further embodiments of the present invention may be devised without departing from the basic scope thereof. It is understood that various embodiments described herein may be utilized in combination with any other embodiment described, without departing from the scope contained herein. Furthermore, the foregoing description is not intended to be exhaustive or to limit the invention to the precise form disclosed. Modifications and variations are possible in light of the above teachings or may be acquired from practice of the invention. Certain exemplary embodiments may be identified by use of an open-ended list that includes wording to indicate that the list items are representative of the embodiments and that the list is not intended to represent a closed list exclusive of further embodiments. Such wording may include "e.g.," "etc.," "such as," "for example," "and so forth," "and the like," etc., and other wording as will be apparent from the surrounding context.

No element, act, or instruction used in the description of the present application should be construed as critical or essential to the invention unless explicitly described as such. Also, as used herein, the article "a" is intended to include one or more items. Where only one item is intended, the term "one" or similar language is used. Further, the terms "any of" followed by a listing of a plurality of items and/or a plurality of categories of items, as used herein, are intended to include "any of," "any combination of," "any multiple of," and/or "any combination of multiples of" the items and/or the categories of items, individually or in conjunction with other items and/or other categories of items.

Moreover, the claims should not be read as limited to the described order or elements unless stated to that effect. In addition, use of the term "means" in any claim is intended to invoke 35 U.S.C. §112, ¶ 6, and any claim without the word "means" is not so intended.

## Claims

1. A mobile device to cleanse data, comprising:
a receiver (311a, 311b) to collect electronic data to cleanse;
a processor (305) coupled to the receiver (311a, 311b), the processor (305) configured to receive the data collected by the receiver and further configured to receive an application container (315) including a first application programming interface (317) and a second application programming interface (323) from a server (303);
a memory (307) coupled to the processor (305), the memory (307) configured to store an application program (309a, 309b), a configuration file and the application container (315) including the first application programming interface (317) and the second application programming interface (323); wherein the configuration file comprises validation rules and data structures and the application container hosts and supports the usage of several application configurations;
wherein the first interface (317) provides programmatic access to an instantiation module (207), the instantiation module (207) is used by the application program (309a, 309b) to build in memory (307) objects required by the first interface (317), the application program (309a, 309b) being operable to use discovery functionality of the instantiation module (207) to learn a data structure of collected data according to a configuration provided by the first interface (317);
wherein the second interface (323) provides programmatic access to a configuration manager module (213), the configuration manager module (213) configured to update the data structures and the validation rules of the configuration file, wherein the configuration file is used by the instantiation module to process data, and wherein the source code of the application program is unchanged,
wherein the first interface (317) and the second interface (323) are callable from the application program (309a, 309b) to cleanse the data collected by the receiver; whereby cleansing of data includes the identification and correction or removal of identified data.

2. The mobile device of claim 1, wherein the instantiation module (207) and the configuration manager module (213) are hosted by the mobile device.

3. The mobile device of claim 1, wherein at least one of the instantiation module (207) and the configuration manager module (213) are hosted externally to the mobile device.

4. The mobile device of claim 1, wherein the first interface (317) and the second interface (323) comprise a respective application program interface (API).

5. The mobile device of claim 1, further comprising a structure manager module (209) coupled to the instantiation module (207), the structure manager module (209) configured to validate the objects.

6. The mobile device of claim 5, further comprising a rules manager module (211) coupled to the instantiation module (207), the rules manager module (211) configured to validate data processed by the instantiation module (207), by use of rules to compare one datum processed by the instantiation module (207) relative to another datum.

7. The mobile device of claim 6, wherein the structure manager module (209) and the rules manager module (211) are configurable without change to the application program (309a, 309b).

8. The mobile device of claim 7, wherein configuration changes to at least one of the structure manager module (209) and the rules manager module (211) take effect without restarting application program (309a, 309b).

9. The mobile device of claim 7, wherein configuration changes to at least one of the structure manager module (209) and the rules manager module (211) are determined algorithmically.

10. The mobile device of claim 6, wherein the structure manager module (209) and the rules manager module (211) operate substantially in real time to validate data.

11. A method to cleanse data, comprising:
providing a mobile device (301) comprising a processor (305) coupled to a memory (307), the processor (305) configured to receive an application container (315) including a first application programming interface (317) and a second application programming interface (323) from a server (303), the memory (307) configured to store an application program (309a, 309b), a configuration file and an application container (315) including the first application programming interface (317) and the second application programming interface (323), wherein the configuration file comprises validation rules and data structures and the application container hosts and supports the usage of several application configurations;
collecting, by a receiver (311a, 311b) coupled to a processor (305), electronic data to cleanse;
wherein the first interface (317) provides programmatic access to an instantiation module (207); the instantiation module (207) is used by the application program (309a, 309b) to build in memory (307) objects required by the first interface (317), the application program (309a, 309b) being operable to use discovery functionality of the instantiation module (207) to learn a data structure of collected data according to a configuration provided by the first interface (317);
updating the data structures and the validation rules of the configuration file, by use of the second interface to execute a configuration manager module by the processor, wherein the configuration file is used by the instantiation module to process data, and wherein the source code of the application program is unchanged,
wherein the first interface and the second interface are callable from the application program (39a, 309b) to cleanse the data collected by the receiver (311a, 311b); whereby cleansing of data includes the identification and correction or removal of identified data.

12. The method of claim 11, wherein the instantiation module and the configuration manager module are hosted by the mobile device.

13. The method of claim 11, wherein at least one of the instantiation module (207) and the configuration manager module are hosted externally to the mobile device.

14. The method of claim 11, wherein the step of processing data collected by the receiver comprises validating the objects.

15. The method of claim 14, wherein the step of processing data collected by the receiver (311a, 311b) further comprises validating data by use of rules to compare one datum relative to another datum.

## Patentansprüche

1. Mobile Vorrichtung zum Bereinigen von Daten, umfassend:
einen Empfänger (311a, 311b) zum Sammeln von elektronischen Daten zum Bereinigen;
einen Prozessor (305), der an den Empfänger (311a, 311b) gekoppelt ist, wobei der Prozessor (305) dazu konfiguriert ist, die durch den Empfänger gesammelten Daten zu empfangen, und ferner dazu konfiguriert ist, einen Anwendungscontainer (315), der eine erste Anwendungsprogrammierschnittstelle (317) und eine zweite Anwendungsprogrammierschnittstelle (323) beinhaltet, von einem Server (303) zu empfangen;
einen Speicher (307), der an den Prozessor (305) gekoppelt ist, wobei der Speicher (307) dazu konfiguriert ist, ein Anwendungsprogramm (309a, 309b), eine Konfigurationsdatei und
den Anwendungscontainer (315), der die erste Anwendungsprogrammierschnittstelle (317) und die zweite Anwendungsprogrammierschnittstelle (323) beinhaltet, zu speichern; wobei die Konfigurationsdatei Validierungsregeln und Datenstrukturen umfasst und der Anwendungscontainer die Nutzung einiger Anwendungskonfigurationen hostet und unterstützt;
wobei die erste Schnittstelle (317) einen programmgesteuerten Zugriff auf ein Instanziierungsmodul (207) bereitstellt, wobei das Instanziierungsmodul (207) durch das Anwendungsprogramm (309a, 309b) verwendet wird, um im Speicher (307) Objekte aufzubauen, die durch die erste Schnittstelle (317) benötigt werden, wobei das Anwendungsprogramm (309a, 309b) dazu dient, eine Discovery-Funktionalität des Instanziierungsmoduls (207) zu verwenden, um eine Datenstruktur gesammelter Daten gemäß einer Konfiguration, die durch die erste Schnittstelle (317) bereitgestellt wird, in Erfahrung zu bringen;
wobei die zweite Schnittstelle (323) einen programmgesteuerten Zugriff auf ein Konfigurationsmanagermodul (213) bereitstellt, wobei das Konfigurationsmanagermodul (213) dazu konfiguriert ist, die Datenstrukturen und die Validierungsregeln der Konfigurationsdatei zu aktualisieren, wobei die Konfigurationsdatei durch das Instanziierungsmodul verwendet wird, um Daten zu verarbeiten, und wobei der Quellcode des Anwendungsprogramms unverändert ist,
wobei die erste Schnittstelle (317) und die zweite Schnittstelle (323) von dem Anwendungsprogramm (309a, 309b) aufrufbar sind, um die durch den Empfänger gesammelten Daten zu bereinigen;
wobei das Bereinigen der Daten die Identifizierung und Korrektur oder Entfernung von identifizierten Daten beinhaltet.

2. Mobile Vorrichtung nach Anspruch 1, wobei das Instanziierungsmodul (207) und das Konfigurationsmanagermodul (213) durch die mobile Vorrichtung gehostet werden.

3. Mobile Vorrichtung nach Anspruch 1, wobei mindestens eines von dem Instanziierungsmodul (207) und dem Konfigurationsmanagermodul (213) außerhalb der mobilen Vorrichtung gehostet wird.

4. Mobile Vorrichtung nach Anspruch 1, wobei die erste Schnittstelle (317) und die zweite Schnittstelle (323) eine jeweilige Anwendungsprogrammschnittstelle (application program interface - API) umfassen.

5. Mobile Vorrichtung nach Anspruch 1, ferner umfassend ein Strukturmanagermodul (209), das an das Instanziierungsmodul (207) gekoppelt ist, wobei das Strukturmanagermodul (209) dazu konfiguriert ist, die Objekte zu validieren.

6. Mobile Vorrichtung nach Anspruch 5, ferner umfassend ein Regelmanagermodul (211), das an das Instanziierungsmodul (207) gekoppelt ist, wobei das Regelmanagermodul (211) dazu konfiguriert ist, durch das Instanziierungsmodul (207) verarbeitete Daten durch Verwendung von Regeln zu validieren, um einen durch das Instanziierungsmodul (207) verarbeiteten Bezugswert bezogen auf einen anderen Bezugswert zu vergleichen.

7. Mobile Vorrichtung nach Anspruch 6, wobei das Strukturmanagermodul (209) und das Regelmanagermodul (211) ohne Änderung am Anwendungsprogramm (309a, 309b) konfigurierbar sind.

8. Mobile Vorrichtung nach Anspruch 7, wobei Konfigurationsänderungen an mindestens einem von dem Strukturmanagermodul (209) und dem Regelmanagermodul (211) ohne Neustarten des Anwendungsprogramms (309a, 309b) in Kraft treten.

9. Mobile Vorrichtung nach Anspruch 7, wobei Konfigurationsänderungen an mindestens einem von dem Strukturmanagermodul (209) und dem Regelmanagermodul (211) algorithmisch bestimmt werden.

10. Mobile Vorrichtung nach Anspruch 6, wobei das Strukturmanagermodul (209) und das Regelmanagermodul (211) im Wesentlichen in Echtzeit arbeiten, um Daten zu validieren.

11. Verfahren zum Bereinigen von Daten, umfassend:
Bereitstellen einer mobilen Vorrichtung (301), umfassend einen Prozessor (305), der an einen Speicher (307) gekoppelt ist, wobei der Prozessor (305) dazu konfiguriert ist, einen Anwendungscontainer (315), der eine erste Anwendungsprogrammierschnittstelle (317) und eine zweite Anwendungsprogrammierschnittstelle (323) beinhaltet, von einem Server (303) zu empfangen, wobei der Speicher (307) dazu konfiguriert ist, ein Anwendungsprogramm (309a, 309b), eine Konfigurationsdatei und einen Anwendungscontainer (315), der die erste Anwendungsprogrammierschnittstelle (317) und die zweite Anwendungsprogrammierschnittstelle (323) beinhaltet, zu speichern, wobei die Konfigurationsdatei Validierungsregeln und Datenstrukturen umfasst und der Anwendungscontainer die Nutzung einiger Anwendungskonfigurationen hostet und unterstützt;
Sammeln von elektronischen Daten zum Bereinigen durch einen Empfänger (311a, 311b), der an einen Prozessor (305) gekoppelt ist;
wobei die erste Schnittstelle (317) einen programmgesteuerten Zugriff auf ein Instanziierungsmodul (207) bereitstellt; wobei das Instanziierungsmodul (207) durch das Anwendungsprogramm (309a, 309b) verwendet wird, um im Speicher (307) Objekte aufzubauen, die durch die erste Schnittstelle (317) benötigt werden, wobei das Anwendungsprogramm (309a, 309b) dazu dient, eine Discovery-Funktionalität des Instanziierungsmoduls (207) zu verwenden, um eine Datenstruktur gesammelter Daten gemäß einer Konfiguration, die durch die erste Schnittstelle (317) bereitgestellt wird, in Erfahrung zu bringen;
Aktualisieren der Datenstrukturen und der Validierungsregeln der Konfigurationsdatei durch Verwendung der zweiten Schnittstelle, um ein Konfigurationsmanagermodul durch den Prozessor auszuführen, wobei die Konfigurationsdatei durch das Instanziierungsmodul verwendet wird, um Daten zu verarbeiten, und wobei der Quellcode des Anwendungsprogramms unverändert ist, wobei die erste Schnittstelle und die zweite Schnittstelle von dem Anwendungsprogramm (39a, 309b) aufrufbar sind, um die durch den Empfänger (311a, 311b) gesammelten Daten zu bereinigen;
wobei das Bereinigen der Daten die Identifizierung und Korrektur oder Entfernung von identifizierten Daten beinhaltet.

12. Verfahren nach Anspruch 11, wobei das Instanziierungsmodul und das Konfigurationsmanagermodul durch die mobile Vorrichtung gehostet werden.

13. Verfahren nach Anspruch 11, wobei mindestens eines von dem Instanziierungsmodul (207) und dem Konfigurationsmanagermodul außerhalb der mobilen Vorrichtung gehostet wird.

14. Verfahren nach Anspruch 11, wobei der Schritt des Verarbeitens von Daten, die durch den Empfänger gesammelt werden, Validieren der Objekte beinhaltet.

15. Verfahren nach Anspruch 14, wobei der Schritt des Verarbeitens von Daten, die durch den Empfänger (311a, 311b) gesammelt werden, ferner Validieren der Daten durch Verwendung von Regeln umfasst, um einen Bezugswert bezogen auf einen anderen Bezugswert zu vergleichen.

## Revendications

1. Dispositif portable de nettoyage de données, comprenant :
un récepteur (311a, 311b) pour collecter des données électroniques à nettoyer ;
un processeur (305) couplé au récepteur (311a, 311b), le processeur (305) étant configuré pour recevoir les données collectées par le récepteur et configuré en outre pour recevoir un conteneur d'application (315) comprenant une première interface de programmation d'application (317) et une seconde interface de programmation d'application (323) en provenance d'un serveur (303) ;
une mémoire (307) couplée au processeur (305), la mémoire (307) étant configurée pour stocker un programme d'application (309a, 309b), un fichier de configuration et le conteneur d'application (315) comprenant la première interface de programmation d'application (317) et la seconde interface de programmation d'application (323) ; dans lequel le fichier de configuration comprend des règles de validation et des structures de données et le conteneur d'application héberge et prend en charge l'utilisation de plusieurs configurations d'application ;
dans lequel la première interface (317) fournit un accès par programme à un module d'instanciation (207), le module d'instanciation (207) est utilisé par le programme d'application (309a, 309b) pour construire en mémoire (307) des objets requis par la première interface (317), le programme d'application (309a, 309b) pouvant fonctionner pour utiliser une fonctionnalité de découverte du module d'instanciation (207) afin d'apprendre une structure de données des données collectées selon une configuration fournie par la première interface (317) ;
dans lequel la seconde interface (323) fournit un accès par programme à un module de gestion de configuration (213), le module de gestion de configuration (213) étant configuré pour mettre à jour les structures de données et les règles de validation du fichier de configuration, dans lequel le fichier de configuration est utilisé par le module d'instanciation pour traiter les données, et dans lequel le code source du programme d'application est inchangé,
dans lequel la première interface (317) et la seconde interface (323) peuvent être appelées depuis le programme d'application (309a, 309b) pour nettoyer les données collectées par le récepteur ; moyennant quoi le nettoyage des données comprend l'identification et la correction ou la suppression des données identifiées.

2. Dispositif portable selon la revendication 1, dans lequel le module d'instanciation (207) et le module de gestion de configuration (213) sont hébergés par le dispositif portable.

3. Dispositif portable selon la revendication 1, dans lequel au moins l'un du module d'instanciation (207) et du module de gestion de configuration (213) est hébergé à l'extérieur du dispositif portable.

4. Dispositif portable selon la revendication 1, dans lequel la première interface (317) et la seconde interface (323) comprennent une interface de programmation d'application (API) respective.

5. Dispositif portable selon la revendication 1, comprenant en outre un module de gestion de structure (209) couplé au module d'instanciation (207), le module de gestion de structure (209) étant configuré pour valider les objets.

6. Dispositif portable selon la revendication 5, comprenant en outre un module de gestion des règles (211) couplé au module d'instanciation (207), le module de gestion des règles (211) étant configuré pour valider les données traitées par le module d'instanciation (207) à l'aide des règles pour comparer une donnée traitée par le module d'instanciation (207) à une autre donnée.

7. Dispositif portable selon la revendication 6, dans lequel le module de gestion de structure (209) et le module de gestion de règles (211) peuvent être configurés sans changer le programme d'application (309a, 309b).

8. Dispositif portable selon la revendication 7, dans lequel les changements de configuration apportés à au moins l'un du module de gestion de structure (209) et du module de gestion de règles (211) prennent effet sans redémarrer le programme d'application (309a, 309b).

9. Dispositif portable selon la revendication 7, dans lequel les changements de configuration apportés à au moins l'un du module de gestion de structure (209) et du module de gestion de règles (211) sont déterminés de manière algorithmique.

10. Dispositif portable selon la revendication 6, dans lequel le module de gestion de structure (209) et le module de gestion de règles (211) fonctionnent sensiblement en temps réel pour valider les données.

11. Procédé de nettoyage de données, comprenant :
la fourniture d'un dispositif portable (301) comprenant un processeur (305) couplé à une mémoire (307), le processeur (305) étant configuré pour recevoir un conteneur d'application (315) comprenant une première interface de programmation d'application (317) et une seconde interface de programmation d'application (323) en provenance d'un serveur (303), la mémoire (307) étant configurée pour stocker un programme d'application (309a, 309b), un fichier de configuration et un conteneur d'application (315) comprenant la première interface de programmation d'application (317) et la seconde interface de programmation d'application (323), dans lequel le fichier de configuration comprend des règles de validation et des structures de données et le conteneur d'application héberge et prend en charge l'utilisation de plusieurs configurations d'application ;
la collecte, par un récepteur (311a, 311b) couplé à un processeur (305), de données électroniques à nettoyer ;
dans lequel la première interface (317) fournit un accès par programme à un module d'instanciation (207) ; le module d'instanciation (207) est utilisé par le programme d'application (309a, 309b) pour construire en mémoire (307) des objets requis par la première interface (317), le programme d'application (309a, 309b) pouvant fonctionner pour utiliser une fonctionnalité de découverte du module d'instanciation (207) afin d'apprendre une structure de données des données collectées selon une configuration fournie par la première interface (317) ;
la mise à jour des structures de données et des règles de validation du fichier de configuration à l'aide de la seconde interface pour exécuter un module de gestion de configuration par le processeur, dans lequel le fichier de configuration est utilisé par le module d'instanciation pour traiter les données, et dans lequel le code source du programme d'application est inchangé,
dans lequel la première interface et la seconde interface peuvent être appelées depuis le programme d'application (39a, 309b) pour nettoyer les données collectées par le récepteur (311a, 311b) ; moyennant quoi le nettoyage des données comprend l'identification et la correction ou la suppression des données identifiées.

12. Procédé selon la revendication 11, dans lequel le module d'instanciation et le module de gestion de configuration sont hébergés par le dispositif portable.

13. Procédé selon la revendication 11, dans lequel au moins l'un du module d'instanciation (207) et du module de gestion de configuration est hébergé à l'extérieur du dispositif portable.

14. Procédé selon la revendication 11, dans lequel l'étape de traitement des données collectées par le récepteur comprend la validation des objets.

15. Procédé selon la revendication 14, dans lequel l'étape de traitement des données collectées par le récepteur (311a, 311b) comprend en outre la validation des données à l'aide des règles pour comparer une donnée à une autre.
